# EUROPÄISCHE PATENTSCHRIFT

(11) **EP 1 100 783 B1**
(45) Veröffentlichungstag und Bekanntmachung des Hinweises auf die Patenterteilung: **23.04.2003**
(21) Anmeldenummer: 99938306.0
(22) Anmeldetag: 21.07.1999
(51) Int. Cl.: C07D 213/89, C07B 41/00

(54) **VERBESSERTES VERFAHREN ZUR HERSTELLUNG VON 2-HALOGENPYRIDIN-N-OXID**
IMPROVED METHOD FOR PRODUCING 2-HALOPYRIDINE-N-OXIDE
AMELIORATION APPORTEE A UN PROCEDE DE PRODUCTION DE N-OXYDE DE 2-HALOPYRIDINE

(30) Priorität: 30.07.1998 DE 19834336
(43) Veröffentlichungstag der Anmeldung: 23.05.2001
(73) Patentinhaber: Rütgers Organics GmbH, 68305 Mannheim (DE)
(72) Erfinder: BARTH, Volker, D-67071 Ludwigshafen (DE); BIEDENBACH, Bruno, D-67547 Worms (DE); HERMES, Karl, D-64625 Bensheim (DE); SENDELBACH, Stefan, D-74172 Neckarsulm (DE); WEINZIERL, Dieter, D-67273 Bobenheim (DE)
(74) Vertreter: Grussdorf, Jürgen, Dr.
(86) Internationale Anmeldenummer: EP9905184
(87) Internationale Veröffentlichungsnummer: WO00006548

(56) Entgegenhaltungen:
- US-A- 3 203 957
- US-A- 4 504 667

## Beschreibung

Gegenstand der vorliegenden Erfindung ist ein verbessertes Verfahren zu Herstellung von 2-Halogenpyridin-N-oxid.

2-Halogenpyridin-N-oxide, insbesondere 2-Chlorpyridin-N-oxid, sind wichtige Zwischenprodukte beispielsweise bei der Herstellung von Pyrithion, insbesondere Zink-Pyrithion, welche als Bakterizide und Fungizide wirksam sind.

Wegen der Elektronen anziehenden Eigenschaften der Halogene lassen sich 2-Halogenpyridine schwieriger oxidieren als andere Pyridinderivate.

Zunächst wurde daher beschrieben, die Oxidation mit Peroxysäuren durchzuführen, wobei jedoch die Umsetzung nur unvollständig ist und ein mehrfaches Recyclisieren des nicht umsetzten Ausgangsmaterials erforderlich wird. Darüber hinaus sind solche Peroxysäuren vergleichsweise teuer und instabil (US-P 2,951,844).

Aus der US-P 4,504,667 ist weiter bekannt, daß 2-Halogenpyridine mittels Peressigsäure mit höherer Ausbeute zu 2-Halogenpyridin-N-oxiden umgesetzt werden können, wenn die Peressigsäure in situ aus Essigsäure und H₂O₂ in Gegenwart eines Katalysators gebildet wird. Als Katalysator werden Maleinsäure, Maleinsäureanhydrid oder Phthalsäureanhydrid in einer Menge von 0,1 - 0,8 Mol/Mol Pyridin verwendet. Nachteilig ist an diesem Verfahren, daß nicht reagierte Halogenpyridine umständlich nach Neutralisation der Lösung durch Wasserdampfdestillation entfernt werden müssen und die zugesetzte Essigsäure und der Katalysator als Salze in der wäßrigen Phase verbleiben und damit verloren gehen.

Eine Verbesserung ist in US-P 3,047,579 beschrieben, wobei 2-Chlorpyridin mit Wasserstoffperoxid in Gegenwart von Perwolframsäure als Katalysator umgesetzt wird. Auch hier ist die Umsetzung mit ca. 68 % unter Verwendung eines Überschusses von H₂O₂ nur unvollständig, erfordert daher eine umständliche Aufarbeitung und Reinigung, und insbesondere eine Wiederaufarbeitung der teueren, umweltschädlichen Schwermetallkatalysatoren.

Einen weiteren wesentlichen Fortschritt zeigt die US-P 3, 203,957, wonach die Oxidation von 2-Chlorpyridin in einem organischen Lösemittel unter Zugabe von wäßrigem 70 %igem Wasserstoffperoxid und Maleinsäureanhydrid durchgeführt wird. Umsätze von über 80 % werden dabei erreicht. Nachteilig an diesem Verfahren ist es, daß das Maleinsäureanhydrid in mindestens äquimolarer Menge bezogen auf das Chlorpyridin eingesetzt werden muß und als Abfallprodukt der Reaktion somit große Mengen Maleinsäure gebildet werden, welche mit Natronlauge aus dem Reaktionsgemisch ausgewaschen werden müssen und entweder als Abfall entsorgt oder aufwendig wieder in Maleinsäureanhydrid zurückverwandelt werden können. Auch die Abtrennung des nicht umgesetzten Halogenpyridins nach diesem Verfahren macht Schwierigkeiten.

Es stellte sich daher die Aufgabe, ein verbessertes Verfahren zur Herstellung von 2-Halogenpyridin-N-oxid, insbesondere von 2-Chlorpyridin-N-oxid zu finden, welches einfach und wirtschaftlich durchzuführen ist.

Die Lösung dieser Aufgabe wird durch die Merkmale des Hauptanspruchs ermöglicht und durch die Merkmale der Unteransprüche gefördert.

Maleinsäureanhydrid und Anthracen lassen sich in einer Diels-Alder-Reaktion zu einer polycyclischen Verbindung (9,10,Dihydro-9,10-ethano-anthracen-11,12-dicarbonsäureanhydrid) der folgenden Formel I 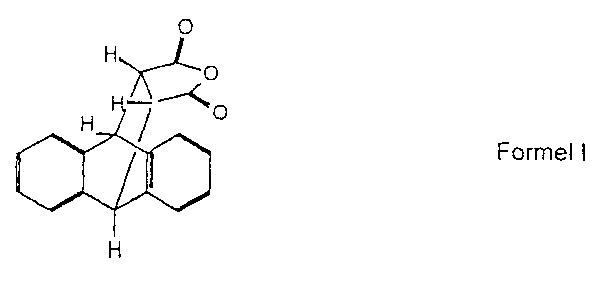 kondensieren (vgl. O. Diels, K. Alder, "Annalen der Chemie" 486, S. 191 - 202 (1931) und E. Clar, Annalen der Chemie 486, 2194 - 2200 (1931)). Dieses Kondensationsprodukt reagiert mit H₂O₂ zu der Peroxysäure der Formel II, 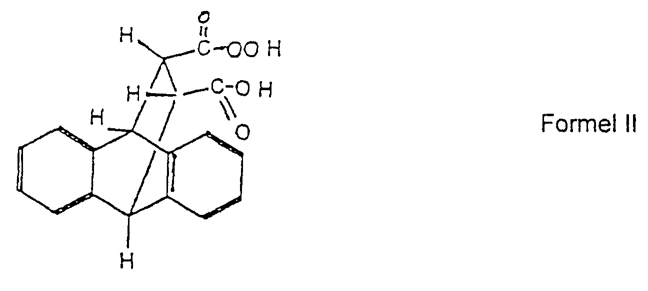 die imstande ist, 2-Halogen-pyridin zum N-Oxid zu oxidieren. Überraschenderweise wird die dabei gebildete Säure der Formel III 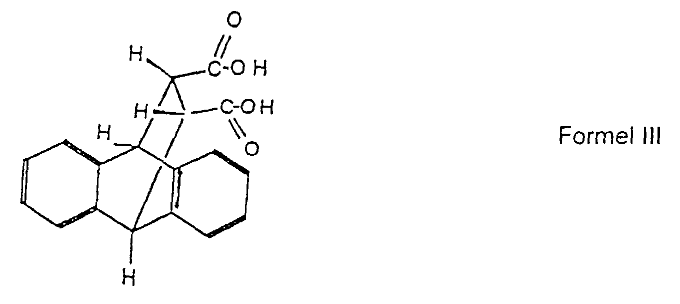 unter den Reaktionsbedingungen trotz des im Überschuß vorhandenen Wassers in das Anhydrid zurückgeführt, so daß katalytische Mengen dieser Verbindung ausreichen, um eine vollständige Reaktion mit dem H₂O₂ zu ermöglichen.

Im Gegensatz dazu erfordern die in der US-P 3,203,957 beschriebenen Reaktionen als Katalysator eine mindestens äquimolare Menge von Maleinsäureanhydrid bzw. Phthalsäureanhydrid, da sich bei der Umsetzung die nicht mehr katalytisch wirkende Maleinsäure bzw. Phthalsäure bildet. Die geringen Mengen des Katalysators der Formel I verbleiben bei der Aufarbeitung des Reaktionsansatze überwiegend in dem nicht umgesetzten 2-Halogenpyridin und können somit immer wieder in die Reaktion zurückgeführt werden. Eine aufwendige Reinigung und Wiedergewinnung des Katalysators entfällt dadurch.

Es wird angenommen, daß anstelle von Anthracen auch andere Anthracenderivate eingesetzt werden können, wie die höheren Polycyclen Naphthacen oder Benz(a)anthracen sowie in den äußeren Ringsystemen durch Alkyl oder Halogen substituierte Anthracene, die in gleicher Weise noch mit Maleinsäureanhydrid zu einer Diels-Alder-Reaktion befähigt sind. Diese Kondensationsprodukte werden im folgenden auch als von der Bezeichnung Anhydrid I mit umfaßt angesehen. Entscheidend erscheint, daß die Rückbildung des Anhydrids I aus der Säure über die sterische Fixierung der benachbarten Kohlenstoffe begünstigt ist, wodurch ein Einsatz von lediglich katalytischen Mengen dieser Verbindung möglich ist und andererseits der große aromatische Rest die Löslichkeit dieser Verbindung in der organischen Phase bewirkt, so daß die Katalysatorverbindung bei der Aufreinigung von 2-Chlorpyridin-N-oxid zurückbleibt. Weniger gehinderte Diels-Alder-Produkte, beispielsweise das aus Butadien und MA gebildete Cyclohexen-4,5-dicarbonsäureanhydrid oder das mit Cyclopentadien gebildete cis-5-Norbornenendo-2,3-dicarbonsäureanhydrid scheinen zur intermediären Rückbildung des Anhydrids nicht befähigt zu sein, da die Umsetzung mit Wasserstoffperoxid nach Verbrauch des Katalysators abbricht.

Eine bevorzugte Verfahrensvariante besteht darin, 2-Chlorpyridin mit katalytischen Mengen des Anhydrids I, beispielsweise 1 bis 25 %, vorzugsweise 10 bis 15 %, zu mischen und dieser Mischung bei Temperaturen zwischen 50 und 100 °C, vorzugsweise etwa 70 bis 80 °C, wäßriges Wasserstoffperoxid unter Rühren zuzufügen. Als Wasserstoffperoxid wird eine handelsübliche 50 bis 70 %ige Lösung verwendet. Nach vollständiger Zugabe des Wasserstoffperoxids, z.B. in einer Zeit von 0,5 h, wird im allgemeinen noch etwa 1 bis 3 h bei der Reaktionstemperatur gehalten, danach abgekühlt und die Reaktionsphase mit Wasser extrahiert. Die Wasserphase wird in an sich bekannter Weise auf 2-Halogenpyridin-N-oxid aufgearbeitet. Die organische Phase, die neben nicht umgesetztem Halogenpyridin geringe Mengen Halogenpyridin-N-oxid und den Katalysator enthält, wird in den nächsten Ansatz zurückgeführt, so daß insoweit Verluste nicht eintreten. Nach Zusatz von frischem 2-Halogenpyridin kann diese Phase für Folgereaktionen ohne weitere Reinigung direkt wieder eingesetzt werden.

Die Reaktion findet normalerweise ohne Zusatz von Lösemitteln statt, da das eingesetzte 2-Chlorpyridin diesen Zweck ausreichend mit erfüllt, jedoch ist ein Zusatz eines inerten Lösungsmittels möglich, beispielsweise eines Chlorkohlenwasserstoffes wie Methylenchlorid, eines Paraffinkohlenwasserstoffes wie Cyclohexan oder n-Heptan, um den Reaktionsansatz zu verdünnen und bei der anschließenden Extraktion mit Wasser die Phasentrennung zu fördern.

Da der Katalysator mit Wasser nicht hydrolysiert wird, ist es möglich, daß die Reaktionsmischung anfänglich bereits Wasser enthält, beispielsweise aus dem vorhergehenden Reaktionszyklus, wobei ca. 3 - 5 % Wasser in der organischen Phase bleiben, oder durch direkten Zusatz von 3 - 20 % Wasser. Ansonsten wird das Wasser, das mit dem Wasserstoffperoxid zugeführt wird, in der warmen Reaktionslösung ohne Bildung einer zweiten Phase gelöst. Eine wäßrige Phase wird erst in der Kälte durch Zusatz von Wasser, beispielsweise 25 - 100 % der Mischung gebildet. Größere Mengen sind nicht sinnvoll, da die Aufarbeitungskosten steigen, kleinere Mengen führen zu einer langsameren Phasentrennung. Da sich das gebildete Halogenpyridin-N-oxid durch Extraktion in Wasser von nicht umgesetztem Halogenpyridin abtrennen läßt, ist es im Sinne einer wirtschaftlichen Umsetzung vorteilhaft, mit einem Unterschuß an Wasserstoffperoxid zu arbeiten. Wasserstoffperoxid in einer Menge von 0,5 bis einem Äquivalent werden dabei bevorzugt. Da bekanntlich bei der Umsetzung ca. 50 % des eingesetzten Peroxids über einen Zerfall in H₂O und O₂ der Reaktion entzogen wird, werden nur 20 - 50 % des eingesetzten Chlorpyridins umgesetzt, der Rest bleibt als Lösemittel für den Katalysator zurück (organische Phase). Andererseits kann natürlich die Reaktion auch durch Zugabe von H₂O₂ im Überschuß im Hinblick auf eine weitgehend quantitative Umsetzung gesteuert werden. Je nachdem werden hohe Ausbeuten in Bezug auf das eingesetzte Peroxid bzw. das eingesetzte Pyridinderivat erhalten.

### Beispiel 1

Anwendungsbeispiel: Herstellvorschrift für 2-Chlorpyridin-N-oxid:
a) Start der Reaktion
   Zu 454 g 2-Chlorpyridin gibt man 65 g Anhydrid 1 und erwärmt auf 80° C. Hierzu tropft man innerhalb von 2 Stunden 140 g 70%iges Wasserstoffperoxid. Anschließend läßt man 3 Stunden bei dieser Temperatur nachreagieren. Danach kühlt man ab und extrahiert dreimal mit jeweils 180 g Wasser. Die Wasserphasen werden vereinigt und gehen in die Herstellung von 2-Mercaptopyridin-N-oxid. Die Ausbeute beträgt 150 g 2-Chlorpyridin-N-oxid. Die organische Phase (304 g Chlorpyridin, 5 g Chlorpyridin-N-oxid, 20 g H₂O, 64 g Anhydrid I) wird für die nächste Oxidation wiederverwendet.
b) Weiteroxidation der organischen Phase
   Die organische Phase aus vorstehender Vorschrift wird mit 150 g frischem 2-Chlorpyridin versetzt. Anschließend erhitzt man auf 80° C und verfährt wie oben beschrieben weiter. Ausbeute 2-Chlorpyridin-N-oxid 155 g.
   Nach 10 Zyklen beträgt der Gesamtumsatz bezogen auf das eingesetzte Anhydrid 1 (von dem noch ca. 55 - 58 g enthalten sind) 4500 %.

### Beispiel 2

### Anwendungsbeispiel: Herstellung von 2-Chlorpyridin-N-oxid (Anwesenheit von Wasser zu Beginn der Reaktion)

Zu 454 g 2-Chlorpyridin gibt man 65 g Anhydrid I und 18 g Wasser und erwärmt auf 80° C. Hierzu tropft man innerhalb von 2 Stunden 140 g 70%iges Wasserstoffperoxid. Anschließend läßt man 3 Stunden bei dieser Temperatur nachreagieren. Danach kühlt man ab und extrahiert dreimal mit jeweils 180 g Wasser. Die Wasserphasen werden vereinigt und gehen in die Herstellung von 2-Mercaptopyridin-N-oxid. Die Ausbeute beträgt 155 g 2-Chlorpyridin-N-oxid. Die organische Phase wird für die nächste Oxidation verwendet.

### Beispiel 3

Zusatz eines organischen Lösungsmittels bei der Aufarbeitung
a) Die erste Herstellung von 2-Chlorpyridin-N-oxid erfolgte gemäß Beispiel 1. Vor der Ausrührung mit Wasser wurden 100 g 1,2-Dichlorethan zugesetzt. Die Phasentrennung war schneller beendet als ohne Dichlorethanzusatz. Die Ausbeute betrug 175 g 2-Chlorpyridin-N-oxid.
b) Die abgetrennte organische Phase aus vorstehend beschriebenem Beispiel wurde mit 176 g 2-Chlorpyridin versetzt und auf die bekannte Art und Weise erneut zu 2-Chlorpyridin-N-oxid umgesetzt. Das in der organischen Phase enthaltene 1,2-Dichlorethan ändert die Reaktion nicht im Vergleich zu Beispiel 1 b). Die Phasentrennung beim Ausrühren mit Wasser erfolgte nochmals etwas schneller als zuvor. Die Ausbeute betrug 174 g 2-Chlorpyridin-N-oxid.

### Beispiel 4

### Aufarbeitung der wäßrigen Extrakte

Die wäßrigen Extrakte werden normalerweise direkt weiterverarbeitet. Zur Gewinnung des reinen Produkts wird die wäßrige Phase mit Chloroform extrahiert, wobei eine vorhergehende Alkalisierung den Übergang in die organische Phase verbessert. Durch Abdestillieren des Lösemittels und Trocknen des zurückbleibenden 2-Chlorpyridin-N-oxids im Vakuum wird der reine Feststoff erhalten.

### Beispiel 5 (Vergleich)

Verwendung alternativer Katalysatoren
a) Verwendung von cis-5-Norbornen-endo-2,3-dicarbonsäureanhydrid (Diels-Alder-Cycloaddukt aus Cyclopentadien und Maleinsäureanhydrid)
   24 g (0,15 mol) Katalysator in 289 g 2-Chlorpyridin wurden mit 89 g Wasserstoffperoxid wie in den vorstehenden Anwendungsbeispielen umgesetzt. Ausbeute 28,4 g verunreinigtes 2-Chlorpyridin-N-oxid.
b) Verwendung von cis-4-Cyclohexen-1,2-dicarbonsäureanhydrid (Diels-Alder-Addukt aus Butadien und Maleinsäureanhydrid)
   Molverhältnisse und Ansatzgröße wie zuvor. Der Versuch ergab große Mengen an nicht umgesetztem Wasserstoffperoxid. Die Ausbeute an 2-Chlorpyridin-N-oxid betrug nur 10 g verunreinigtes 2-Chlorpyridin-N-oxid.

## Patentansprüche

1. Verfahren zur Herstellung von 2-Halogenpyridin-N-oxid durch Umsetzen von 2-Halogenpyridin mit einer 50 bis 70%igen wäßrigen Wasserstoffperoxidlösung in Gegenwart eines Säureanhydridkatalysators bei Temperaturen von 50° C bis 100° C, **dadurch gekennzeichnet, daß** als Katalysator ein Anhydrid der Formel I 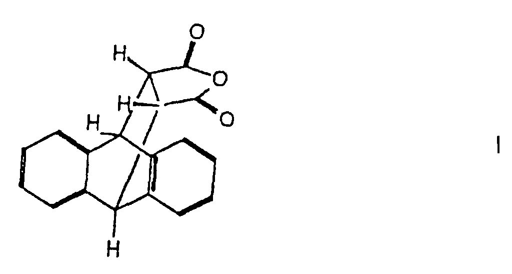 verwendet wird, wobei die aromatischen Ringe durch Alkylgruppen oder Halogene substituiert oder mit einem weiteren aromatischen Ring anelliert sein können, wobei bezogen auf das 2-Halogenpyridin
a) das Anhydrid in einer Menge von 1 bis 25 % und
b) das Wasserstoffperoxid in einer Menge mindestens 0,5 Äquivalent eingesetzt wird.

2. Verfahren gemäß Anspruch 1, **dadurch gekennzeichnet, daß** bei der Reaktion zusätzlich ein inertes Lösemittel verwendet wird.

3. Verfahren gemäß den Ansprüchen 1 bis 2, **dadurch gekennzeichnet, daß** das entstandene 2-Halogenpyridin-N-oxid durch Extraktion mit Wasser aus der Reaktionslösung abgetrennt und die nicht wäßrige Phase für den nächsten Reaktionsansatz eingesetzt wird.

4. Verfahren gemäß den Ansprüchen 1 bis 3, **dadurch gekennzeichnet, daß** der Reaktionsmischung aus Halogenpyridin und Katalysator vor der Zugabe von Wasserstoffperoxid bis zu 20 % Wasser zugefügt wird.

5. Verfahren gemäß den Ansprüchen 1 bis 4, **dadurch gekennzeichnet, daß** H₂O₂ in einer Menge von bis zu 1 Äquivalent bezogen auf das 2-Halogenpyridin eingesetzt wird.

6. Verfahren gemäß den Ansprüchen 1 bis 5, **dadurch gekennzeichnet, daß** als 2-Halogenpyridin 2-Chlorpyridin eingesetzt wird.

## Claims

1. Process for the preparation of 2-halopyridine N-oxide by reaction of 2-halopyridine with a 50 to 70% aqueous hydrogen peroxide solution in the presence of an acid anhydride catalyst at temperatures of 50°C to 100°C, **characterised in that**, as catalyst, there is used an anhydride of the formula I 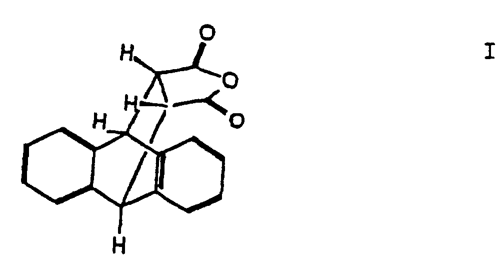 whereby the aromatic rings can be substituted by alkyl groups or halogens or can be anellated with a further aromatic ring, whereby, referred to the 2-halopyridine
a) the anhydride is used in an amount of 1 to 25% and
b) the hydrogen peroxide is used in an amount of at least 0.5 equivalents.

2. Process according to claim 1, **characterised in that**, in the case of the reaction, an inert solvent is additionally used.

3. Process according to claims 1 to 2, **characterised in that** the resulting 2-halopyridine N-oxide is separated from the reaction solution by extraction with water and the non-aqueous phase is used for the next reaction batch.

4. Process according to claims 1 to 3, **characterised in that** to the reaction mixture of halopyridine and catalyst, up to 20% of water is added before the addition of hydrogen peroxide.

5. Process according to claims 1 to 4, **characterised in that** H₂O₂ is used in an amount of up to 1 equivalent, referred to the 2-halopyridine.

6. Process according to claims 1 to 5, **characterised in that** 2-chloropyridine is used as 2-halopyridine.

## Revendications

1. Procédé de préparation de N-oxyde de 2-halogénopyridine par réaction de 2-halogénopyridine avec une solution aqueuse de peroxyde d'hydrogène à 50 à 70 % en présence d'un catalyseur constitué d'un anhydride d'acide à des températures de 50°C à 100°C, **caractérisé en ce qu'**on utilise comme catalyseur un anhydride répondant à la formule I 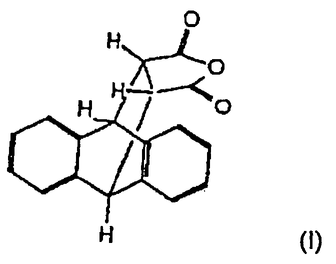 les noyaux aromatiques pouvant être substitués par des groupes alkyles ou des halogènes ou condensés avec un noyau aromatique supplémentaire, en utilisant, par rapport à la 2-halogénopyridine,
a) l'anhydride dans une proportion de 1 à 25 % et
b) le peroxyde d'hydrogène en une quantité d'au moins 0,5 équivalent.

2. Procédé selon la revendication 1, **caractérisé en ce qu'**on utilise en outre dans la réaction un solvant inerte.

3. Procédé selon les revendications 1 à 2, **caractérisé en ce que** le N-oxyde de 2-halogénopyridine formé est séparé de la solution de réaction par extraction avec de l'eau, et la phase non aqueuse est utilisée pour l'étape de réaction suivante.

4. Procédé selon les revendications 1 à 3, caractérisé en qu'on ajoute au mélange réactionnel constitué d'halogénopyridine et de catalyseur jusqu'à 20 % d'eau avant l'addition du peroxyde d'hydrogène.

5. Procédé selon les revendications 1 à 4, **caractérisé en ce que** H₂O₂ est utilisé en une quantité allant jusqu'à 1 équivalent par rapport à la 2-halogénopyridine.

6. Procédé selon les revendications 1 à 5, **caractérisé en ce qu'**on utilise comme 2-halogénopyridine la 2-chloropyridine.
